# EUROPEAN PATENT SPECIFICATION

(11) **EP 1 133 259 B1**
(45) Date of publication and mention of the grant of the patent: **22.09.2004**
(21) Application number: 99950608.2
(22) Date of filing: 05.10.1999
(51) Int. Cl.: A61B 10/00, A61B 19/00

(54) **BIOPSY SYSTEM**
BIOPSIESYSTEM
SYSTEME DE BIOPSIE

(30) Priority: 24.11.1998 DE 19855293
(43) Date of publication of application: 19.09.2001
(73) Proprietor: Ethicon Endo-Surgery (Europe) GmbH, 22851 Norderstedt (DE)
(72) Inventor: BILOTTI, Federico, D-22559 Hamburg (DE); REU, Gene, San Diego, CA 92130 (US)
(74) Representative: Both, Georg
(86) International application number: PCT/EP1999/007333
(87) International publication number: WO 2000/030545

(56) References cited:
- WO-A-91/07922
- WO-A-96/24289
- WO-A-97/47243
- DE-A- 3 617 235
- US-A- 4 576 175
- US-A- 5 794 626

## Description

The invention relates to a biopsy system with a biopsy device and a mounting device, as known from WO-A-97/47 243.

Using a biopsy device, a tissue sample can be removed from a patient. For this purpose, a removal instrument is used, for example in the form of a hollow needle, which is brought up to the patient and inserted into the tissue in question. If a hollow needle is used, and the tissue is soft, a tissue sample can be sucked into the hollow needle. This does not constitute a serious operation on the patient. The further treatment of the patient follows from the result from the removed tissue sample.

It is often of great advantage if such a biopsy device is used while observing the removal of tissue with the help of a diagnostic device. An X-ray device comes into consideration in particular as a diagnostic device, but other devices such as for example an ultrasound device are also conceivable. Such diagnostic devices are elaborate and expensive. For this reason, they should be able to be as universally usable as possible. If a particular biopsy device were to be permanently attached in the area of the diagnostic device that was accessible to the patient, the versatility of the diagnostic device would be considerably limited. The latter would then not be available for other equipment which is to be used depending on the application.

The object of the invention is to make it possible to use a biopsy device quickly and safely in conjunction with a given diagnostic device without the space available on the diagnostic device being permanently restricted by the biopsy device.

This object is achieved by a biopsy system with the features of claim 1. Advantageous versions of the invention emerge from the dependent claims.

The biopsy system according to the invention has a biopsy device and a mounting device. The biopsy device is arranged for the removal of a tissue sample from a patient and has a removal instrument and a holding apparatus arranged for mounting the biopsy device on the mounting device. The mounting device can be attached to a diagnostic device, in particular an X-ray device, and is adapted to mount the biopsy device. A rectangular-like recess provided on the mounting device has two inner wide sides facing each other and two inner narrow sides facing each other. A two-armed swivel lever with a first lever arm and a second lever arm is pre-tensioned by a spring into a locking position in which the first lever arm projects into the recess. The first lever arm runs sloped on its outer side which projects into the recess and is set up to swivel in against the action of the spring when an object matched to the cross-section of the recess is inserted into the recess. Opposite the sloped outer side of the first lever arm, the second lever arm has a projection designed as a pushbutton, which is accessible from an outer side of the mounting device and with whose help the swivel lever can be swivelled in against the action of the spring into an unlocking position.

Once the mounting device is attached to a desired diagnostic device, it can be used repeatedly to mount the biopsy device quickly if the biopsy device is required. The biopsy device is held secure by the mounting device and can thus be used in a reliable and reproducible manner under the control of the diagnostic device.

In a preferred version of the invention, the holding apparatus of the biopsy device has a holding projection which can be inserted in the rectangular-like recess provided on the mounting device. The holding projection of the biopsy device is preferably rectangular-like and its cross-section matched to the cross-section of the recess on the mounting device. The holding projection of the biopsy device preferably has a recess, and the first lever arm of the swivel lever is set up to project in locking engagement into the recess on the holding projection when the biopsy device is in the mounted state in locking position of the swivel lever. This version has the particular advantage that the biopsy device can be connected to the mounting device with one grip. To do this, the holding projection of the biopsy device is inserted into the rectangular-like recess of the mounting device. As soon as the free end of the holding projection engages with the sloped outer side of the first lever arm of the swivel lever, it pushes the first lever arm against the action of the spring out of the free cross-section of the recess (i.e. the swivel lever is swivelled in), until the first lever arm lies opposite the recess at the holding projection. At this point, the holding projection preferably strikes a stop in the mounting device. In any case, the swivel lever then swivels into the locking position in which the first lever arm engages in the recess in the holding projection. Thus, the biopsy device is secured immovably at the mounting device. The release from locking is also effected very easily. To do this, it is necessary only to press the pushbutton accessible from an outer side of the mounting device, the swivel lever thereby swivelling into the unlocking position. Subsequently, the biopsy device can be withdrawn from the rectangular-like recess of the mounting device.

In an advantageous design of the invention, the mounting device has a guide apparatus which is set up to guide the removal instrument of the biopsy device when the biopsy device is mounted. For example, the guide apparatus can have a guide bar with a guide opening which surrounds the removal instrument of the biopsy device when the biopsy device is mounted. In particular with needle-like removal instruments which have only a limited flexural strength, the removal instrument can be guided more precisely using the guide apparatus when removing tissue. If the removal instrument in the biopsy device is mounted in a displaceable manner, the removal instrument can glide in the guide opening so that the guide apparatus can be stationary relative to the rest of the mounting device and the other parts of the biopsy device.

Preferably, the guide apparatus is removable from the rest of the mounting device. For this, the guide apparatus can, for example, have a mandrel which has a slit-like recess transverse to its longitudinal axis and can be inserted into a recess on the mounting device matched to its cross-section. The angled end of a locking spring projects into this recess, which end, if the mandrel is inserted into this recess, engages in the slit-like recess of the mandrel. With this design, the guide apparatus can be connected to the mounting device or removed from it with one grip.

In a preferred version, there is provided in the entry zone of the rectangular-like recess of the mounting device a step against which a housing part for the biopsy device rests when the biopsy device is mounted. This step provides additional grip, in particular if it runs largely horizontally so that it can support part of the weight of the biopsy device.

In the following, the invention is explained in more detail with reference to an embodiment. The diagrams show in
- Figure 1: a perspective view of an embodiment of a biopsy system according to the invention with a biopsy device which has a holding projection, and with a mounting device,
- Figure 2: an exploded view of the mounting device according to Figure 1 in which the component featuring the holding projection of the biopsy device is additionally drawn in,
- Figure 3: the arrangement from Figure 2 in the assembled state in perspective view and
- Figure 4: a longitudinal section through the elements essential in the embodiment according to Figure 2 for mounting the biopsy device on the mounting device.

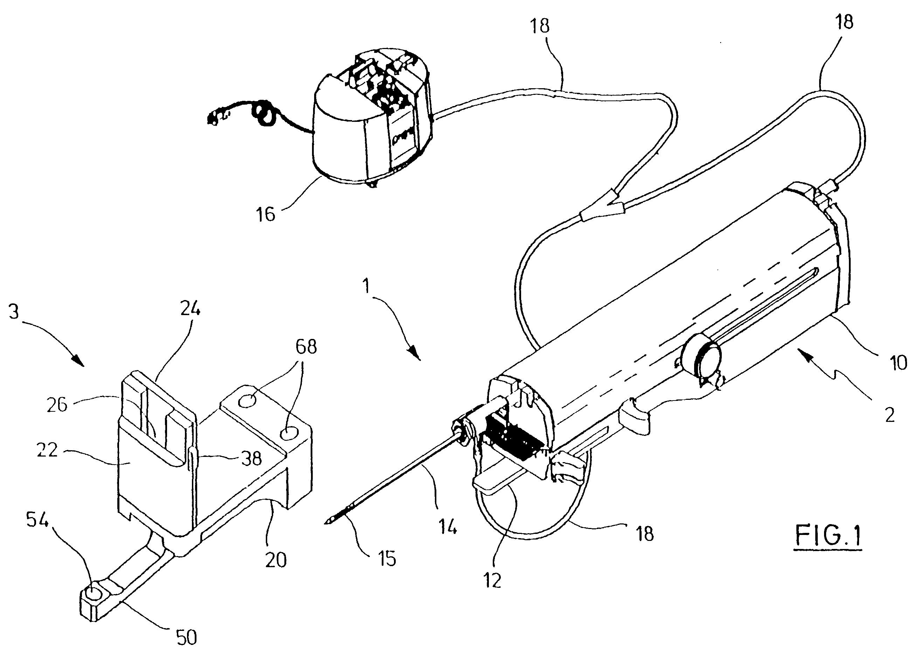

In Figure 1, an embodiment of a biopsy system 1 is shown in perspective view. The biopsy system 1 has a biopsy device 2 and a mounting device 3.

The biopsy device 2 has a housing 10. At one end of the housing 10, a holding projection 12 projects sideways which is rigidly anchored to the supporting parts of the biopsy device 2. A removal instrument 14 extends parallel to the holding projection 12. In the embodiment, the removal instrument 14 is designed as a hollow needle with a solid point and a side opening in the point zone 15 and can be rotated about its longitudinal axis using the operating elements of the biopsy device 2. A pump 16 is connected to the housing 10 of the biopsy device 2 via tubes 18. The pump 16 creates a vacuum which can be used to suck in tissue samples through the opening in the point zone 15 of the removal instrument 14. Apart from the holding projection 12, the biopsy device 2 is of conventional design.

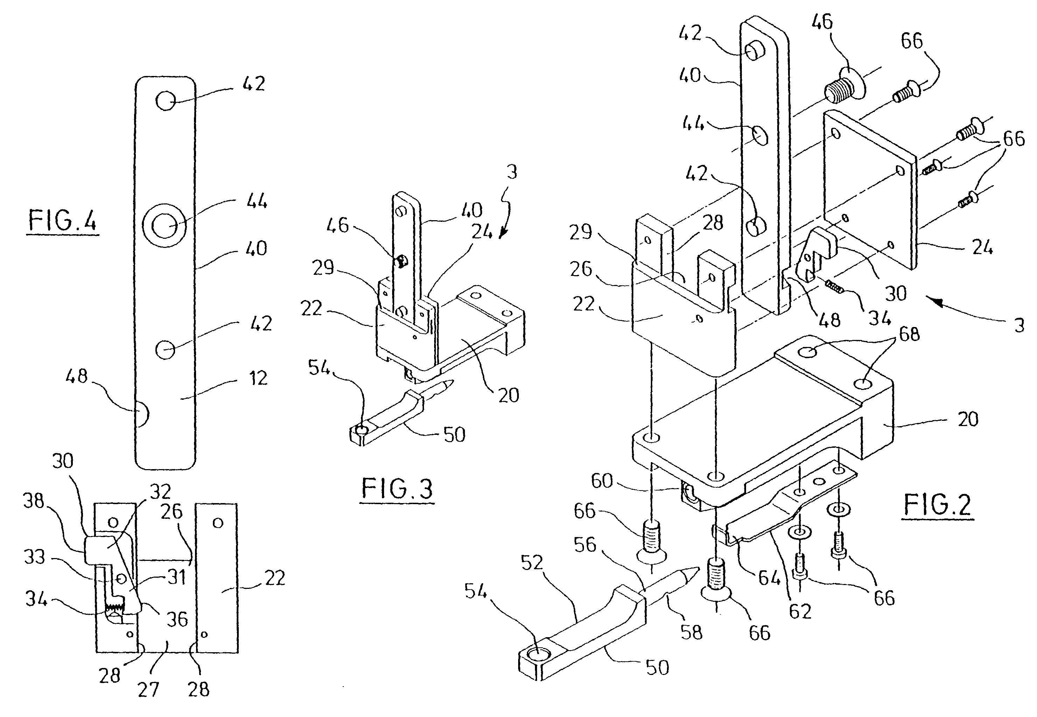

The mounting device 3 is represented in Figure 2 in exploded view to show the individual components clearly. The component of the biopsy device 2 which features the holding projection 12 is also shown in Figure 2. The arrangement from Figure 2 is shown assembled in Figure 3.

The mounting device 3 has a base plate 20 from which (with the orientation according to Figure 2) a front part 22 and a back plate 24 extend upwards. Between the front part 22 and the back plate 24, an essentially rectangular recess 26 is formed which is limited at the bottom by the base plate 20 and has two wide inner sides 27 facing each other (on the front part 22 and back plate 24) and two narrow inner sides 28 facing each other (on both side zones of the front part 22). As the extension of the front side of the front part 22, measured in a direction perpendicular to the base plate 20, is smaller than the extension of the back plate 24, a step 29 is formed on the front part 22. The step 29 thus lies in the freely accessible entry zone of the recess 26.

Fitted in a side recess of the front part 22 is a swivel lever 30, see in particular Figure 4, which is a longitudinal section running through the recess 26 in a plane parallel to the back plate 24. The swivel lever 30 is two-armed with a first lever arm 31 and a second lever arm 32. The fulcrum is situated at a pivot pin 33 which is inserted into the front part 22 through a bore which can be recognised in Figure 2; the pivot pin 33 is not shown directly in Figure 2. A helical spring 34 biases the swivel lever 30 into a locking position in which the first lever arm 31 projects into the recess 26, see Figure 4. On its outer side 36 which projects into the recess, the first lever 31 runs obliquely, i.e. when in the locking position it extends at a relatively flat angle relative to the longitudinal axis of the recess 26 which runs perpendicular to the base plate 20. Vis-à-vis the sloped outer side 36 of the first lever arm 31, the second lever arm 32 has a projection which is designed as pushbutton 38 and projects outwards from the recess in the front part 22. By pressing on the pushbutton 38, the swivel lever 30 can be swivelled from the locking position according to Figure 4 into an unlocking position in which it no longer projects into the recess 26.

The holding projection 12 of the biopsy device 2 is the end zone of a bar 40 which is shown as an individual component in Figures 2 to 4. Two projecting pegs 42, which engage in two holes in the biopsy device 2 and provide a safeguard against turning, and a countersunk screw 46, inserted through a sunk bore 44, which is screwed into a threaded hole in the biopsy device 2, serve to anchor the bar 40 securely to supporting parts of the biopsy device 2. The cross-section of the holding projection 12 is matched to the cross-section of the recess 26 so that when the holding projection 12 is inserted in the recess 26, the biopsy device 2 is mounted securely and secure against turning on the mounting device 3.

In the zone of the holding projection 12, the bar 40 is provided with a recess 48, see also Figure 4. If the holding projection 12 is pushed into the recess 26 from above according to the representation in Figure 4, its bottom end glides along the outer side 36 of the first lever arm 31 and, in doing so, presses the swivel lever 30 into the unlocking position. The holding projection 12 can be advanced until its bottom end strikes the base plate. The recess 48 is then opposite the first lever arm 31, and the first lever arm 31 can return unhindered back into the locking position under the action of the helical spring 34. In doing this, it engages in the recess 48. If there is now an upward pull on holding projection 12, the swivel lever 30 remains in the locking position because of the design of the outer side 36 of its first lever arm 31. The holding projection 12 (and thus the biopsy device 2) can thus be pushed into the recess 26 with one grip and is locked immediately.

To release from locking, the pushbutton 38 must be pressed, thus moving the swivel lever 30 into its unlocking position. The biopsy device 2 with the holding projection 12 can then be pulled out upwards without problems.

To achieve a better grip for the removal instrument 14, which has a limited flexural strength, of the biopsy device 2, the mounting device 3 is provided with a guide apparatus 50, see Figures 1 to 3. The guide apparatus 50 has a guide bar 52 which runs essentially perpendicular to the removal instrument 14 when the biopsy device 2 is in the mounted position, and on one end of which a guide opening 54 is located through which the removal instrument 14 can be inserted. At the opposite end, a mandrel 56 extends from the guide bar 52. Transverse to its longitudinal axis, the mandrel 56 has a slit-like recess 58, see in particular Figure 2. The mandrel 56 can be inserted into a recess 60, matched to its diameter, on the bottom side of the base plate 20 of the mounting device 3. If it is completely pushed through, a locking spring 62 with an angled end 64 ensures that it can be pulled out of the recess 60 again only by exerting a considerable amount of force, as the angled end 64 of the locking spring 62 engages in the slit-like recess 58. The guide apparatus 50 can thus be easily attached to the rest of the mounting device 3 and removed from it again. It need only be inserted if it is also actually required for a given biopsy device, and does not therefore interfere when the mounting device 3 is otherwise used.

The parts drawn in Figure 2 can be connected to each other using the screws 66. The screws 66 are of varying sizes and engage in each case in threaded holes. Bores 68 in the base plate 20, through which securing bolts can be inserted, serve to mount the mounting device 3 permanently on a diagnostic device.

The way in which the biopsy system 1 is used has been largely shown already in the foregoing. The mounting device 3 is fixedly screwed to the desired diagnostic device (an X-ray device, for example). In principle, the orientation of the mounting device 3 is as desired. Permanent mounting on a swivellable component of the diagnostic device is also conceivable. To connect a desired biopsy device 2 to the mounting device 3, the holding projection 12 of the biopsy device 2 is pushed into the recess 26 via the freely accessible end of the recess 26 until the swivel lever 30 is in the locking position again. Then the biopsy device 2 is securely and immovably connected to the mounting device 3. In the embodiment, the housing 10 rests against the step 29 when the biopsy device 2 is in this mounted state. As a result, at least as long as the step 29 runs largely horizontally, the stability of the arrangement is further increased. The biopsy device 2 can now be used on a patient 2 in the desired way. This can be monitored in a safe way using the diagnostic device, an X-ray device in the embodiment, as there are no undesired movements thanks to the secure mounting of the biopsy device 2 on the mounting device 3 and thus on the diagnostic device. In the embodiment, the removal instrument 14 is introduced into the tissue for example to remove mammary tissue whilst under the supervision of the diagnostic device and brought to a desired point. To do this, the removal instrument 14 can be rotated about its longitudinal axis so that the opening in the point zone 15 reaches a suitable tissue point. By applying below-atmospheric pressure using the pump 16, tissue can be sucked into the biopsy device 2.

If the biopsy device 2 on the diagnostic device is no longer required, the release from locking can be effected by pressing the pushbutton 38, and the biopsy device 2 with the holding projection 12 can be withdrawn without problems from the recess 26.

## Claims

1. Biopsy system,
- with a biopsy device (2) and
- with a mounting device (3),
- the biopsy device (2) being arranged for the removal of a tissue sample from a patient, and having a removal instrument (14) and a holding apparatus (12) arranged for mounting the biopsy device (2) on the mounting device (3), **characterised by**
- the mounting device (3) being able to be attached to a diagnostic device, in particular an X-ray device, the mounting device (3) being arranged for mounting the biopsy device (2), and the mounting device (3) having a rectangular-like recess (26) with two inner wide sides (27) facing each other and two inner narrow sides (28) facing each other, the mounting device (3) furthermore having a two-armed swivel lever (30) with a first lever arm (31) and a second lever arm (32), the swivel lever (30) being pre-tensioned by a spring (34) into a locking position in which the first lever arm (31) projects into the recess (26), the first lever arm (31) running sloped on its outer side (36) which projects into the recess (26) and being set up so that it swivels in against the action of the spring (34) when an object matched to the cross-section of the recess (26) is inserted into the recess (26), and the second lever arm (32) having, opposite the sloped outer side (36) of the first lever arm (31), a projection constructed as a pushbutton (38) which is accessible from an outer side of the mounting device (3) and by means of which the swivel lever (30) can be swivelled in against the action of the spring (34) into an unlocking position.

2. Biopsy system according to claim 1, **characterized in that** the holding apparatus of the biopsy device (2) has a holding projection (12) which can be inserted into the rectangular-like recess (26) provided on the mounting device (3).

3. Biopsy system according to claim 2, **characterized in that** the holding projection (12) of the biopsy device (2) is rectangular-like and is matched in cross-section to the cross-section of the recess (26) of the mounting device (3).

4. Biopsy system according to claim 2 or 3, **characterized in that** the holding projection (12) of the biopsy device (2) has a recess (48) and the first lever arm (31) of the swivel lever (30) is set up to project in locking engagement into the recess (48) on the holding projection (12) when the biopsy device (2) is in the mounted state in locking position of the swivel lever (30).

5. Biopsy system according to one of claims 1 to 4, **characterized in that** the mounting device (3) has a guide apparatus (50) which is set up to guide the removal instrument (14) of the biopsy device (2) when the biopsy device (2) is mounted.

6. Biopsy system according to claim 5, **characterized in that** the guide apparatus (50) has a guide bar (52) with a guide opening (54) which surrounds the removal instrument (14) of the biopsy device (2) when the biopsy device (2) is mounted.

7. Biopsy system according to claim 5 or 6, **characterized in that** the guide apparatus (50) is removable from the rest of the mounting device (3).

8. Biopsy system according to claim 7, **characterized in that** the guide apparatus (50) has a mandrel (56) which has a slit-like recess (58) transverse to its longitudinal axis, the mandrel (56) being insertable into a further recess (60) on the mounting device (3) matched to fit its cross-section, an angled end (64) of a locking spring (62) projecting into said further recess (60), said angled end (64), if the mandrel (56) is inserted into said recess (60), engages in the slit-like recess (58) of the mandrel (56).

9. Biopsy system according to one of claims 1 to 8, **characterized in that** there is provided in an entry zone of the rectangular-like recess (26) of the mounting device (3) a step (29) against which a housing part of the biopsy device (2) rests when the biopsy device (2) is mounted.

## Patentansprüche

1. Biopsiesystem,
- mit einer Biopsievorrichtung (2) und
- mit einer Montagevorrichtung (3),
- wobei die Biopsievorrichtung (2) zur Entnahme einer Gewebeprobe von einem Patienten eingerichtet ist und ein Entnahmeinstrument (14) und eine zur Montage der Biopsievorrichtung (2) an der Montagevorrichtung (3) eingerichtete Halteeinrichtung (12) aufweist,
**dadurch gekennzeichnet, daß**
- die Montagevorrichtung (3) an einer Diagnostikvorrichtung, insbesondere einer Röntgenvorrichtung, anbringbar ist, wobei die Montagevorrichtung (3) zur Montage der Biopsievorrichtung (2) eingerichtet ist und wobei die Montagevorrichtung (3) eine quaderähnliche Aussparung (26) mit zwei sich gegenüberliegenden inneren Breitseiten (27) und zwei sich gegenüberliegenden inneren Schmalseiten (28) aufweist, wobei die Montagevorrichtung (3) ferner einen zweiarmigen Schwenkhebel (30) mit einem ersten Hebelarm (31) und einem zweiten Hebelarm (32) aufweist, wobei der Schwenkhebel (30) von einer Feder (34) in eine Arretierstellung vorgespannt ist, in der der erste Hebelarm (31) in die Aussparung (26) hineinragt, wobei der erste Hebelarm (31) an seiner in die Aussparung (26) hineinragenden Außenseite (36) abgeschrägt verläuft und dazu eingerichtet ist, beim Einstecken eines an den Querschnitt der Aussparung (26) angepaßten Gegenstands in die Aussparung (26) entgegen der Wirkung der Feder (34) einzuschwenken, und wobei der zweite Hebelarm (32) gegenüber der abgeschrägten Außenseite (36) des ersten Hebelarms (31) einen als Drucktaste (38) ausgebildeten Vorsprung aufweist, der von einer Außenseite der Montagevorrichtung (3) zugänglich ist und mittels dem der Schwenkhebel (30) entgegen der Wirkung der Feder (34) in eine Entarretierstellung einschwenkbar ist.

2. Biopsiesystem nach Anspruch 1, **dadurch gekennzeichnet, daß** die Halteeinrichtung der Biopsievorrichtung (2) einen Haltevorsprung (12) aufweist, der in die an der Montagevorrichtung (3) vorgesehene quaderähnliche Aussparung (26) einsteckbar ist.

3. Biopsiesystem nach Anspruch 2, **dadurch gekennzeichnet, daß** der Haltevorsprung (12) der Biopsievorrichtung (2) quaderähnlich ist und im Querschnitt an den Querschnitt der Aussparung (26) der Montagevorrichtung (3) angepaßt ist.

4. Biopsiesystem nach Anspruch 2 oder 3, **dadurch gekennzeichnet, daß** der Haltevorsprung (12) der Biopsievorrichtung (2) eine Ausnehmung (48) aufweist und der erste Hebelarm (31) des Schwenkhebels (30) dazu eingerichtet ist, im montierten Zustand der Biopsievorrichtung (2) in Arretierstellung des Schwenkhebels (30) in arretierendem Eingriff in die Ausnehmung (48) an dem Haltevorsprung (12) hineinzuragen.

5. Biopsiesystem nach einem der Ansprüche 1 bis 4, **dadurch gekennzeichnet, daß** die Montagevorrichtung (3) eine Führungseinrichtung (50) aufweist, die im montierten Zustand der Biopsievorrichtung (2) zum Führen des Entnahmeinstruments (14) der Biopsievorrichtung (2) eingerichtet ist.

6. Biopsiesystem nach Anspruch 5, **dadurch gekennzeichnet, daß** die Führungseinrichtung (50) einen Führungsriegel (52) mit einer Führungsöffnung (54) aufweist, die im montierten Zustand der Biopsievorrichtung (2) das Entnahmeinstrument (14) der Biopsievorrichtung (2) umgibt.

7. Biopsiesystem nach Anspruch 5 oder 6, **dadurch gekennzeichnet, daß** die Führungseinrichtung (50) von der übrigen Montagevorrichtung (3) abnehmbar ist.

8. Biopsiesystem nach Anspruch 7, **dadurch gekennzeichnet, daß** die Führungseinrichtung (50) einen Dorn (56) aufweist, der quer zu seiner Längsachse eine schlitzähnliche Ausnehmung (58) hat, wobei der Dorn (56) in eine an seinen Querschnitt angepaßte weitere Ausnehmung (60) an der Montagevorrichtung (3) einsetzbar ist, wobei in diese weitere Ausnehmung (60) ein abgewinkeltes Ende (64) einer Arretierfeder (62) hineinragt, wobei dieses abgewinkelte Ende (64) bei in diese Ausnehmung (60) eingesetztem Dorn (56) in die schlitzähnliche Ausnehmung (58) des Dorns (56) eingreift.

9. Biopsiesystem nach einem der Ansprüche 1 bis 8, **dadurch gekennzeichnet, daß** in einem Eingangsbereich der quaderähnlichen Aussparung (26) der Montagevorrichtung (3) eine Stufe (29) vorgesehen ist, an der im montierten Zustand der Biopsievorrichtung (2) ein Gehäuseteil der Biopsievorrichtung (2) anliegt.

## Revendications

1. Système de biopsie,
- avec un dispositif de biopsie (2) et
- avec un dispositif de montage (3),
- le dispositif de biopsie (2) étant conçu pour le retrait d'un échantillon de tissu d'un patient et possédant un instrument de retrait (14) et un appareil de support (12) conçus pour monter le dispositif de biopsie (2) sur le dispositif de montage (3),
**caractérisé en ce que**
- le dispositif de montage (3) peut être fixé à un dispositif de diagnostic, en particulier un dispositif à rayons X, le dispositif de montage (3) étant conçu pour monter le dispositif de biopsie (2) et le dispositif de montage (3) possédant un évidement de type rectangulaire (26) doté de deux côtés intérieurs larges (27) face à face et deux côtés intérieurs étroits (28) face à face, le dispositif de montage (3) comprenant, en outre, un levier pivotant à deux bras (30) avec un premier bras de levier (31) et un second bras de levier (32), le levier pivotant (30) étant prétendu par un ressort (34) dans une position de verrouillage dans laquelle le premier bras de levier (31) dépasse à l'intérieur de l'évidement (26), le premier bras de levier (31) étant incliné sur son bord extérieur (36) qui déoasse à l'intérieur de l'évidement (26) et étant monté de façon à pivoter vers l'intérieur en réponse à l'action du ressort (34) lorsqu'un objet de même coupe transversale que l'évidement (26) est inséré dans l'évidement (26), lé second bras de levier (32) ayant, sur le bord opposé au bord extérieur incliné (36) du premier bras de levier (31), une avancée conçue comme un bouton-poussoir (38) qui est accessible depuis un bord extérieur du dispositif de montage (3) et au moyen duquel le levier pivotant (30) peut être pivoté vers l'intérieur en réponse à l'action du ressort (34) pour atteindre une position de déverrouillage.

2. Système de biopsie selon la revendication 1, **caractérisé en ce que** l'appareil de support du dispositif de biopsie (2) possède une avancée de support (12) qui peut être insérée dans l'évidement de type rectangulaire (26) prévu sur le dispositif de montage (3).

3. Système de biopsie selon la revendication 2, **caractérisé en ce que** l'avancée de support (12) du dispositif de biopsie (2) est de type rectangulaire et de même coupe transversale que celle de l'évidement (26) du dispositif de montage (3).

4. Système de biopsie selon la revendication 2 ou 3, **caractérisé en ce que** l'avancée de support (12) du dispositif de biopsie (2) possède un évidement (48) et **en ce que** le premier bras de levier (31) du levier pivotant (30) est monté pour dépasser en position de verrouillage à l'intérieur de l'évidement (48) sur l'avancée de support (12) lorsque le dispositif de biopsie (2) est dans l'état monté en position de verrouillage du levier pivotant (30).

5. Système de biopsie selon l'une des revendications 1 à 4, **caractérisé en ce que** le dispositif de montage (3) possède un appareil de guidage (50) qui est prévu pour guider l'instrument de retrait (14) du dispositif de biopsie (2) lorsque le dispositif de biopsie (2) est monté.

6. Système de biopsie selon la revendication 5, **caractérisé en ce que** l'appareil de guidage (50) possède une barre de guidage (52) dotée d'une ouverture de guidage (54) qui entoure l'instrument de retrait (14) du dispositif de biopsie (2) lorsque le dispositif de biopsie (2) est monté.

7. Système de biopsie selon la revendication 5 ou 6, **caractérisé en ce que** l'appareil de guidage (50) peut être retiré du reste du dispositif de montage (3).

8. Système de biopsie selon la revendication 7, **caractérisé en ce que** l'appareil de guidage (50) possède un mandrin (56) doté d'un évidement de type fente (58) transversal par rapport à son axe longitudinal, le mandrin (56) pouvant être inséré dans un autre évidement (60) sur le dispositif de montage (3) de coupe transversale identique, une extrémité à angle (64) d'un ressort de verrouillage (62) dépassant à l'intérieur du dit autre évidement (60), ladite extrémité à angle (64) s'engrenant dans l'évidement de type fente (58) du mandrin (56) si le mandrin (56) est inséré dans ledit évidement (60).

9. Système de biopsie selon l'une des revendications 1 à 8, **caractérisé en ce qu'**il est prévu, dans une zone d'entrée de l'évidement de type rectangulaire (26) du dispositif de montage (3), une marche (29) contre laquelle une partie boîtier du dispositif de biopsie (2) reposé lorsque le dispositif de biopsie (2) est monté.
